(19) Europäisches Patentamt / European Patent Office / Office européen des brevets

(11) **EP 4 553 842 A1**

(12) **EUROPEAN PATENT APPLICATION**

(43) Date of publication:
**14.05.2025 Bulletin 2025/20**

(21) Application number: **23208722.1**

(22) Date of filing: **09.11.2023**

(51) International Patent Classification (IPC):
**G16H 15/00** (2018.01)    **G16H 50/20** (2018.01)
**G16H 30/40** (2018.01)

(52) Cooperative Patent Classification (CPC):
**G16H 15/00; G16H 50/20;** G16H 30/40

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(71) Applicant: **Koninklijke Philips N.V.
5656 AG Eindhoven (NL)**

(72) Inventors:
• **WEESE, Rolf Jürgen**
  **Eindhoven (NL)**
• **FLÄSCHNER, Nick**
  **Eindhoven (NL)**
• **WENZEL, Fabian**
  **Eindhoven (NL)**
• **EWALD, Arne**
  **Eindhoven (NL)**

(74) Representative: **Philips Intellectual Property &
Standards
High Tech Campus 52
5656 AG Eindhoven (NL)**

(54) **DETERMINING STRUCTURED MEDICAL FINDINGS IN A MEDICAL REPORT AND ADJUSTING A MEDICAL REPORT**

(57)    A computer-implemented method for determining at least one structured medical finding in a medical report (1), the method comprising the following steps:
(a) receiving the medical report (1), wherein the medical report (1) comprises text segments (6) describing medical findings, and receiving at least one medical image on which the medical report (1) is based;
(b) applying a searching algorithm to the medical report (1) in order to identify medical findings in the medical report (1) and determine corresponding structured medical findings;
(c) at least in case the determination of a structured medical finding by the searching algorithm is fulfils a pre-defined ambiguity criterion, applying a classification algorithm to the medical image to verify and/or complement the structured medical finding, wherein the output of the classification algorithm is at least one probable structured medical finding, and, if applicable, adjusting the structured medical finding determined by the searching algorithm on the basis of the output of the classification algorithm.

FIG. 3

EP 4 553 842 A1

**Description**

FIELD OF THE INVENTION

The invention relates to a computer-implemented method for determining at least one structured medical finding in a medical report, a computer-implemented method for adjusting a medical report and a corresponding computer program and computer system.

BACKGROUND OF THE INVENTION

In the medical context, in particular concerning radiology, reporting is often done by using speech recognition and free text. However, it has been found that the descriptions of medical findings in the resulting text of the medical report may often be unnecessary complicated and/or long, thus making it potentially difficult to read and understand. Additionally, a medical report may often contain inconsistencies or inaccuracies.

Michael P. Hartung, Ian C. Bickle, Frank Gaillard, Jeffrey P. Kanne have proposed how radiology reports should be formulated in principle in "How to Create a Great Radiology Report", RadioGraphics 2020, 40(6), 1658-1670. However, it is questionable, whether rules like these can always be followed accurately by radiologists or other medical staff. Furthermore, mistakes and different approaches how to structure medical reports may still lead to inconsistencies or inaccuracies

OBJECT OF THE INVENTION

It is, therefore, an object of the invention to provide a method to better address the above-mentioned problems. In particular it would be desirable to provide a method or means to automatically adapt existing medical reports to become more efficient, e.g., to become easier to understand, more concise, shorter and/or closer to a standard report format. Furthermore, it would be desirable to find a solution to automatically determine findings in a medical report.

SUMMARY OF THE INVENTION

To address this object, a method according to claim 1, a method according to claim 9, a computer program according to claim 14, and a computer system according to claim 15 is provided. Advantageous embodiments are set out in the dependent claims. Any features, advantages or alternative embodiments described herein in relation to the claimed method are also applicable to the other claim categories and vice versa. The features and advantages of different embodiments as described herein can be combined.

According to a first aspect, a computer-implemented method for determining at least one structured medical finding in a medical report is provided. The method comprises the following steps:

(a) receiving the medical report, wherein the medical report comprises one or several text segments describing medical findings, and receiving at least one medical image on which the medical report is based;
(b) applying a searching algorithm to the medical report in order to identify at least one medical finding in the medical report and determine at least one corresponding structured medical finding;
(c) at least in case the determination of a structured medical finding by the searching algorithm fulfils a pre-defined ambiguity criterion, applying a classification algorithm to the medical image to verify and/or complement the structured medical finding, wherein the output of the classification algorithm is at least one probable structured medical finding, and, if applicable, adjusting the structured medical finding determined by the searching algorithm on the basis of the output of the classification algorithm.

Advantageously, medical findings in the medical report can be identified automatically by the searching algorithm and then be associated with structured findings. Since it may not always be possible to determine the medical findings in the medical report, e.g. due to unclear descriptions or missing information, the classification algorithm may advantageously be used to resolve such ambiguities and/or to provide a higher confidence in actually determine correct structured medical findings.

A medical report is to be understood broadly in the context of this invention. It generally describes any kind of report comprising medical findings. "Medical findings" may also be simply denoted as "findings". Medical findings may describe particular observations, in particular such as deviations or anomalies, typically based on the observation of a medical image. Usually, medical findings are described by text segments. However, further segments, such as drawings or images may also be included. Besides medical findings, a medical report may comprise further information. A typical example of a medical report comprises multiple, preferably at least three, parts. One part is a part describing the medical findings. Another part may comprise general information, such as information about the patient and/or a medical question to be answered, i.e., the reason for the examination. Another part may comprise a diagnosis, such as an interpretation of the medical findings and/or a summary. For example, the medical report may be a radiology report. A radiology report may be a medical report that is based on the output of a radiation-based imaging modality, such as a computed tomography (CT) image. In particular, the medical report may be a medical report that is to be adjusted via this method and/or with the help of this method.

The starting point of the method is in particular the medical report (that is to be adjusted). The invention does not have to be limited to a particular way of receiving the medical report. The medical report may, for example, be received by downloading or otherwise transmitting it from an external source, such as an external database, or from an internal database. For example, the medical report may be obtained from at least one of EHR (Electronic Health Report), EMR (Electronic Medical Record), HIS (Health Information System), RIS (Radiology Information System). The medical report may be input by a user. For example, a user interface may be provided that allows a user to input the medical report and/or to provide an address, such as a web link, from where the medical report can be retrieved. Optionally, the medical report may be provided by the user by writing the medical report or by dictating a corresponding text that is transformed into text by a speech recognition software. The medical report comprises one or several text segments describing medical findings. Text segments may, for example be or comprise sentences, partial sentences, or paragraphs of text.

The medical image may, for example, be a radiology image, such as a CT image. However, any other kind of medical image, such as a magnetic resonance tomography image, is also conceivable. The medical image may, for example, be a two-dimensional image or a three-dimensional image. The medical image may comprise or be part of a time-series of images. Additionally or alternatively, the medical image may comprise or be part of more than one image acquisition (e.g. acquisitions with different contrasts in magnetic resonance tomography). The medical report is based on the medical image. This statement is to be understood broadly. The medical report may be based directly or indirectly on the medical image. For example, the medical report may be based directly on the medical image by a user studying the medical image and drafting the medical report based on observations on the medical image. An example of the medical report being indirectly based on the medical image may be a user studying another image derived from the medical image and drafting the medical report based on observations on the other image. Alternatively, the user may have drafted the medical report based on another medical image that shows the same or equivalent area of the same patient as the medical image. Hence, for example, one medical image may be used by the user for drafting the report and another, equivalent, medical image may be received in the context of this method. Preferably, the medical image the medical report is based on is the same medical image that is received during this method. In this case, it may be more likely that the medical findings derivable from the medical image are actually the same as the ones that are or should be in the medical report. However, other variants may also have advantages. For example, one medical image may be more suitable for a user to study in order to write the medical report and another medical image may be more suitable for the classification algorithm to determine structured medical findings. Optionally, multiple medical images may be provided. Multiple medical images may for example comprise medical images depicting different points of views of the same anatomic position. Multiple medical images may, for example, comprise medical images from different imaging modalities.

In a simple variant, the searching algorithm may be a word searching algorithm that is looking for specific text segments, in particular keywords, in the text segments. Examples of specific text segments may be abbreviations, parts of sentences, etc. Keywords may be words that are typically used to describe a particular medical finding. A medical finding may be associated with a list of keywords, such that the presence of all or most of the list's keywords indicates the medical finding. The searching algorithm may be configured to have synonyms of keywords. Hence, despite the application of different synonyms in different medical reports, the searching algorithm may still be able to determine medical findings. Optionally, a more complex algorithm, such as a machine learning algorithm that is trained to recognize medical findings, may be applied. Optionally, the searching algorithm may comprise or be applied together with an optical character recognition (OCR) algorithm. The OCR algorithm may be applied when the medical report is available in image form, e.g. a scan of a physical medical report. Hence, the OCR algorithm may be applied to transform a non-text format of the medical report into a text format. The identification of the at least one medical finding can be used to determine at least one corresponding structured medical finding. Hence, in other words, the searching algorithm may be configured to recognize structured medical findings in the medical report by associating the text segments of the medical report with the structured medical findings. The searching algorithm may link the corresponding text segments of the medical report to the structured medical findings. A structured medical finding may be a medical finding that is structured according to a standardized structure. For example, the standardized structure may comprise a standardized description of a location and/or of an anatomical part. The standardized structure may comprise a standardized description for an anomaly. Examples are given in table 1. For example, an anomaly may comprise a grading such as Stoller 1. For example, a location and/or anatomical part may comprise a compartment such as "femoro-tibial". The standardized structure may comprise standardized paragraphs and/or standardized subheadings. The standardized structure may be standardized according to propositions given in the state of the art, in particular in literature such as cited above. For example, the searching algorithm may comprise a database of structured medical findings or have access to a database of structured medical findings.

The determination of a structured medical finding may be ambiguous and thus fulfil the ambiguity criterion, if information concerning the medical finding in the medical report is not suitable to clearly determine a structured medical finding. For example, the determination of a structured medical finding may be ambiguous, if information concerning the structured medical finding is missing in the medical report or if parts of the medical report provide contradictory information. The ambiguity criterion may be missing information concerning the structured medical finding and/or contradictory information in the medical report concerning the structured medical finding. Missing information concerning the structured medical finding may, for example, be a missing property that is not defined in the medical report. The missing property may be added by the result of the classification algorithm. Hence, the found properties of the structured finding may be complemented with additional properties provided by the classification algorithm. Complementing may comprise identifying a structured medical finding, e.g. from multiple image-based classifiers, that is in agreement with the given medical finding, e.g. with given properties of the given medical finding, and/or is determined to have the largest probability to be correct, e.g. to describe the same medical finding. Contradictory information may, for example, be at least one part of a text segment of the medical report that points to the finding in question being a first medical finding and another part of the text segment that points to the finding in question being a second medical finding. In this case, the classification algorithm may be used to verify which of the first and second medical findings is most likely the correct one. Verifying by the classification algorithm may comprise assessing the image-based probability of the medical finding to be correct. For example, if the probability is large, e.g. close to 100% and/or above a defined threshold, the medical finding may be considered to be correct, and thus be verified. For example, if the probability is low, e.g. close to 0% and/or below a defined threshold, the medical finding may be considered to be incorrect.

According to the invention, the classification algorithm is applied if the determination of the structured medical finding is ambiguous. Hence, advantageously, the classification algorithm may be used to resolve the ambiguity. On the other hand, the step of applying the classification algorithm may be a conditional step that is not applied when the output of the searching algorithm is non-ambiguous. For example, non-ambiguity may be assumed when a text segment, such as a sentence, uniquely defines a medical finding. Optionally, the classification algorithm may also be applied when further criteria are fulfilled. One example for a further criterium may be a user request, requesting the application of the classification algorithm. Optionally, the classification algorithm may be applied every time. This may include cases without ambiguity. The classification algorithm may thus be used to always verify the finding of the searching algorithm.

The output of the classification algorithm is at least one probable structured medical finding. In particular the output may be the structured medical finding that is determined by the classification algorithm to be most likely correct. The classification algorithm may be a trained machine learning algorithm, such as a trained neural network. Adjusting the structured medical finding may be applicable depending on the output of the classification algorithm. For example, adjusting may be applicable if the probable structured finding output by the classification algorithm differs from the structured medical finding determined by the searching algorithm. For example, adjusting may be applicable if the structured medical finding is not verified, i.e. is determined to be incorrect. For example, adjusting may be applicable the classification algorithm finds that the structured medical finding should be complemented. Adjusting the structured medical finding may comprise complementing the structured medical finding. Complementing may comprise adding missing parts of the structured medical finding, such as one or several missing properties. Additionally or alternatively, adjusting the structured medical finding may comprise correcting the structured medical finding, e.g. by replacing a part of the structure of the structured medical finding, such as a property, by another part as determined by the classification algorithm. The classification algorithm may comprise multiple classifiers. The classifiers may be individually configured to detect specific medical findings and/or specific anatomical parts. For example, when each classifier is associated with all properties defining a medical finding, missing properties may be derived from a classifier that is identified to be most likely in agreement with a medical finding of the medical report.

The structured medical finding may be assigned to the corresponding text segment, such as to a sentence or a section of the medical report. The output of the method may be one or several structured medical findings that are assigned to corresponding one or several text segments.

According to an embodiment, the classification algorithm comprises an anatomical segmentation model that segments image information in the medical image into anatomical parts, wherein the classification algorithm detects and determines probable structured medical findings based on the segmented anatomical parts. For example, an image of the knee may be segmented into the knee's parts, such as knee cap, cartilage, meniscus, sinews, and others. The anatomical segmentation model may preferably be a trained machine learning algorithm. For training the anatomical segmentation model medical images acquired according to clinical routine standards may preferably be used. This may ensure that the training data resemble realistic image data from clinical examinations. For example, for training (e.g. for anatomical segmentation) 100 image data sets may be sufficient. Optionally, in particular to train classifiers for medical findings, more datasets might be used. While this test concerns knee examinations, the invention is generally suitable for other anatomical areas, as well. The at least one anatomical segmentation model may be a model-based segmentation (MBS) model. The geometrical representation of the MBS model may be an average triangular surface mesh, derived from a set of manually segmented volumes. For example, the mean mesh of the model may be automatically positioned in the medical image. For example, the positioning may be performed according to a localization technique known as the Generalized Hough Transform (GHT). Starting from the initialized mesh, structure boundaries close to the mesh surface may be detected by evaluating an individual boundary detector for each mesh triangle. For each triangle, a cuboid grid of data values around the triangle normal may be sampled and a convolutional neural network may return (i.e. predict) a regression value that estimates the signed distance of the target point along the triangle normal. For model adaptation, an optimization problem for the external energy that penalizes squared distances of triangle centres from target planes that are orthogonal to the local image gradient passing through the target points may be applied. Weights may be used to reflect the reliability of a detected boundary and be considered as constants during energy optimization. The weights, may, for example, be derived from gradient information. The mesh may be transformed, and the process may be iterated by alternating target point detection and energy minimization until convergence. A global affine transformation may account for potential scaling or shearing of anatomical structures. The anatomical segmentation model being an MBS model can be advantageous, as the pre-defined anatomical knowledge of the MBS model may help localizing anatomical structures even in the absence of clear image features. This may be helpful for small structures such as ligaments or in the case of norm-variants or pathologies such as ligament tears. The pre-defined topology of the model can then be used to point to the area in the image, where the structure is expected based on the localization of the adjacent structures. Alternatively, the anatomical segmentation model may, for example, be a voxel-based model, such as U-Net (see O. Ronneberger, P. Fischer, and T. Brox, "U-Net: Convolutional Networks for Biomedical Image Segmentation," arXiv: 1505.04597 [cs], May 2015. arXiv: 1505.04597), which is incorporated herein by reference. The classification algorithm may comprise a trained machine learning algorithm, such as a neural network (e.g. ResNet) that has been trained for a specific medical finding, in particular for a specific structured finding associated with the anatomical part. Multiple such trained machine learning algorithms may be provided for specific medical findings. The trained machine learning algorithms may be denoted as classifiers.

According to an embodiment, based on the segmented anatomical parts, regions of interest are defined that are most likely to contain medical findings, wherein the regions of interest are used to detect and determine structured medical findings in the medical image. For example, minimum enclosing boxes may be provided to define the regions of interest. The minimum enclosing boxes may provide an additional rim around the segmented anatomical structure, in particular where a medical finding is associated with. The classification algorithm may comprise at least one classifier that is configured to detect and determine the structured medical findings in the medical image based on the regions of interest. The classification algorithm may comprise multiple classifiers, wherein a classifier is specifically trained for a particular region of interest and/or a particular medical finding, respectively. The at least one classifier may comprise a trained machine learning algorithm, in particular a trained neural network (e.g. ResNet). The at least one classifier may be trained with annotated image data comprising image parts showing the particular regions of interest. The annotation may, for example, comprise a binary label. The binary label may be encoded in a vector. The at least one classifier may be trained to recognize structured medical findings in the image parts. The input of the at least one classifier may, for example, be the medical image together with the region of interest. Multiple classifiers may be provided to detect multiple specific medical findings. Each classifier may be associated with and/or trained for a specific medical finding. The segmentation mask may be modified by aggregating multiple integer mask values into one, in order to define the region of interest. Which mask values are aggregated into which target value may, for example, be defined via a dictionary. For example, the dictionary may be build based on rules according to general anatomical knowledge (e.g. rules such as: when "cartilage damage" in the knee is to be assessed, all cartilage labels need to be taken into account for the region of interest).

According to an embodiment, the structured medical findings are structured to comprise multiple properties, at least one of the properties describing an anatomic position of the medical finding and at least one of the properties describing a medical abnormality. Advantageously, the structured medical findings may be structured such that a location of the finding and the kind of finding can be determined. Optionally, one or several of the properties, preferably each property, may comprise a list of options that may apply for that property. The options may be specific for the corresponding property. The list of options may, at least in some cases, consist of only one option. Depending on the type of property, a property may be defined by a single option or by a set of feasible options. The options may in particular be options that can be assigned to each property. For example, in knee-related findings concerning the anterior horn, pars intermedia, or posterior horn of the meniscus, the property "grading" may have the options "Stoller 1", "Stoller 2", "Stoller 3", "Stoller 4", and "Stoller 5". For example, options may be given for a discoid meniscus based according to the known Watanabe classification.

According to an embodiment, the multiple properties comprise a sequence of properties, wherein at least one of the properties determines at least one following property and/or the list of options for a following property in the sequence. In particular, the property-specific options may depend on the option defined for another property. Optionally, at least in some cases, a property may not be used in dependence of selected options for other properties. For example, a sequence of properties may be as shown in table 1.

According to an embodiment, a first one of the properties describes a general anatomic position and a second one of the properties describes a more specific anatomic position depending on the first one of the properties. For example, the first one of the properties may describe a compartment, the second one of the properties may describe a sub-compartment. For example, in knee-related findings, the compartment-property may have the options femoro-tibial or femoro-patellar, and the sub-compartment-property may have the options medial, lateral. A further property may, for example, be the type of structure. In the example of the knee, the type of structure may, for example have the options meniscus or cartilage.

According to an embodiment, the searching algorithm and/or the classification algorithm is configured to determine findings based on the structure of the structured medical findings. For example, the searching algorithm may search for properties according to the structured medical findings in the medical report. For example, the classification algorithm may associate features of the medical image with properties or options of the properties of the structured medical findings. Using the structured medical findings as basis for the searching algorithm and/or the classification algorithm may provide particularly efficient and simple means to determine the findings. The classification algorithm being based on the structure of the structured medical findings may enable an association with the medical finding to be particularly efficient and simple.

The classification algorithm may comprise multiple classifiers. The classifiers may be individually configured to detect specific medical findings. Hence, individual classifiers may be trained to each detect different medical findings. For example, with knee-specific medical reports, one classifier may be configured, to detect cartilage damage, another may be configured to detect meniscus tear, and another may be configured to detect anterior crucial ligament sprain. For example, the classifiers may be based on trained neural networks. For example, a classifier might be trained to predict the grade of the cartilage damage in the central part of the medial femur, i.e. ground truth information of the grade of the cartilage damage in the central part of the medial femur may be used during training within a loss function. Another classifier might be trained to predict the grade of the damage of the anterior-horn of the lateral meniscus by using ground truth information of the grade of the damage of the anterior-horn of the lateral meniscus during training within the loss function.

The properties and/or the options may be complemented with one or multiple synonyms. Hence, for particular terms, synonyms may be provided that describe the properties and/or options. Advantageously, the searching algorithm may thus be more robust against the possibility that medical reports may use different words or term to describe the same property, such as an anatomical position or pathology. The searching algorithm may be configured to identify medical findings based on a keyword search, in particular as described herein.

According to an embodiment, the searching algorithm is configured to search for keywords describing the medical findings in order to identify structured medical findings in the medical report, wherein in particular the keywords are options of properties of the structured medical findings described in text form or synonyms thereof. A keyword search may provide a simple, yet efficient way to look for findings. In particular when combined with the search being based on the structured medical findings, it has been found that a keyword search can work very well. For example, given a text segment, such as a sentence, from the medical report, the searching algorithm may search the options (and optionally corresponding synonyms) for one property after the other in the sentence. The text segment that is searched may comprise, for example, a sentence, a section in the report and/or a sentence together with a section heading that the searched sentence belongs to. For example, a section may concern the femoro-tibial compartment or the femoro-patellar compartment. Optionally, properties and/or options of properties may also be derived from section headings.

According to an embodiment, the searching algorithm is configured to apply a hashing search in order to find and determine the findings. For example, a hash table may be used for the search. The hashing search may be provided analogously to hash searches as known in the state of the art. Alternatively a synonym search may be applied in order to find and determine the findings. The synonym search may comprise a keyword search. Optionally, the hashing search may be used in addition to the synonym search.

According to an embodiment, an ambiguity comprises a missing property of a structured medical finding in the medical report, wherein the classification algorithm is configured to determine and add the missing property to the structured medical finding. A property may, for example, be missing because a user writing the medical report forgot to include the property. The property may also be missing due to the searching algorithm not being able to identify the property. This may, for example, be due to a misspelling (including terms wrongly recognized by a speech recognition program) or the use of one or several synonyms unknown to the searching algorithm. For example, information about a sub-structure of the medical finding or about a subsub-structure may be missing. Advantageously, the classification algorithm may thus be used to determine complementing information of the medical report. The classification algorithm may thus advantageously be applied to determine which structured medical finding is described in the report, even if the medical report does not comprise enough information for such an assertion. The complemented structured medical finding may, for example, be used to ensure that the medical report can be adapted to comprise complete information.

According to an embodiment, the classification algorithm is configured to determine the most likely missing property and to add the most likely missing property to the structured medical finding. In the case of multiple missing properties, the most likely missing property may be determined and added multiple times, in particular for each missing property. The classification algorithm may be based on a trained machine learning algorithm, in particular based on a trained neural-network. In particular a Residual Neural Network (ResNet), as known in the state of the art, may be used.

The machine learning algorithm may be configured to output a classifier that denotes a probability $P(f|I)$. $I$ may denote the image; optionally $I$ may represent multiple images (e.g. from magnetic resonance imaging with different contrasts). $P(f|I)$ is the probability of finding the structured medical finding $f$ given image $I$.

Assuming that one property of a structured medical finding $f(.)$ with options $w_1, ..., w_n$ is missing, e.g. is unidentified, the missing option may, for example, be defined via the max operation

$$w^* = \max_{i} P(f(w_i)|I),$$

where the probability $P(f(w_i)|I)$ describes the probabilities of structured medical findings derived via the classification algorithm. A single classifier or the combination of multiple classifiers may be used in this context. Optionally, an option may be excluded from the max operation. This may, for example, be useful in case that the option $w_j$ corresponds to a previously identified finding (e.g. identified in another text segment of the medical report). The max operation may thus be adapted as follows:

$$w^* = \max_{i; i \neq j} P(f(w_i)|I).$$

The probability as determined may be assigned to the corresponding structured medical finding. This may allow to check the confidence level of the result.

According to an embodiment, at least one identifier is stored together with the medical report that comprises information about the at least one structured medical finding.

For example, the identifier may be a sequence of numbers that define the properties of the medical findings. The numbers may be integer numbers. Preferably, the number of the sequence is a fixed number. The identifier may be configured to look up words representing a property of a medical finding, e.g. in different languages. Advantageously, when storing the identifier, structured medical findings that have previously been identified can be treated as "known" and may thus be used for processing without parsing the text of the medical report again. For example, an identifier analogous to identifiers as typically used in medical ontologies may be used.

According to another aspect of the invention, a computer-implemented method for adjusting a medical report towards a standardized medical report format is provided. The standardized medical report format comprises standardized text segments describing medical findings.

The method comprises the following steps:

- carrying out the method for determining at least one structured medical finding in a medical report as described herein;
- automatically performing or proposing to a user an adjustment of the medical report based on the structured medical finding.

Advantageously, the method can provide a means to associate parts of the medical report, in particular text segments with structured medical findings and accordingly adapt the medical report or propose an adaption of the medical report. The proposing to a user may be provided via a user interface. The user interface may be adapted to allow a user to accept and/or decline the proposed adjustment. The user interface may provide additional information and/or selection options to the user. Text segments may, for example, be or comprise sentences, partial sentences, or paragraphs of text. Hence, standardized text segments may be sentences, partial sentences, or paragraphs that are formulated according to a standard format. For example, pre-formulated sentences, partial sentences, and/or paragraphs may be used that are pre-formulated for specific medical findings. Examples, of how a medical report may be generally adjusted are known in the state of the art. For example, in Michael P. Hartung, Ian C. Bickle, Frank Gaillard, Jeffrey P. Kanne have proposed how radiology reports should be formulated in principle in "How to Create a Great Radiology Report", RadioGraphics 2020, 40(6), 1658-1670 such examples are given. According to this citation, for example, the text "There are likely small pleural effusions on the basis of blunting of the posterior costophrenic angles, which appear new compared to prior" may be adjusted to the standardized text segment "New blunting of the costophrenic angles".

According to an embodiment, the adjustment of the medical report comprises one or more of the following: replacement, addition, and simplification medical report, in particular of the one or several text segments according to the at least one standardized text segment. Simplification may, for example, comprise replacing sentences or parts of sentences with simpler, e.g. less complicated and/or easier to understand, sentences or parts of sentences. Replacement may comprise a correction of the medical report. Correcting may, for example, comprise a formal, e.g. grammatical, correction. Additionally or alternatively, correcting may comprise correction of the content, e.g. a correction of false statements about medical findings. The correction may be based on the application of the classification algorithm. A correction may, for example, be proposed if a finding can completely be identified from the text of the medical report, but image classification by the classification algorithm indicates that the finding is very unlikely and/or has a very low probability and the probability becomes much larger if the correction is applied, e.g. if one property is changed. Hence the correction may comprise changing the corresponding property. Addition may comprise adding missing information, such as missing properties of structured medical findings.

According to an embodiment, the adjustment of the medical report comprises adding a standardized text segment corresponding to a new structured medical finding identified by the classification algorithm. Hence, the classification may optionally be adapted to identify medical findings that may have been overlooked in the original medical report. Hence, optionally, the method may thus also be used as additional verification means for the integrity of the medical report.

According to an embodiment, at least one standardized report structure element corresponding to the structured medical finding is retrieved from a standardized report structure source, wherein performing or proposing to a user an adjustment of the medical report is based on the at least one standardized report structure element. The at least one standardized report structure element may in particular comprise at least one standardized text segment and/or at least one standardized object including graphics. Advantageously, retrieving the standardized report structure element may allow to transform the medical report into a standardized format according to the standardized report structure source. Using the standardized report structure source may thus allow to apply a uniform structure of medical reports. The standardized report structure source may be an internal source, e.g. part of the system or device with which the method is performed. The standardized report structure source may be an external source, e.g. an internet database.

According to an embodiment, the standardized text resource comprises standardized text segments in a plurality of languages for each structured medical finding. Preferably the method may comprise receiving user input regarding a selection of at least one language for the adjustments of the medical report. Advantageously, the report may thus be adapted in multiple languages. Further, a translation functionality may be added, such that the medical report can be translated into another language based on the structured medical findings. Advantageously, using the structured medical findings may allow to provide an accurate translation, wherein the integrity of the meaning of the original medical report can be preserved and the translation can be ensured to be in appropriate standardized language due to the standardization of the structured medical findings.

According to an embodiment, proposing an adjustment to a user comprises marking a report element, in particular a text segment, in the medical report, in particular a text segment corresponding to an ambiguous structured medical finding, and displaying to the user the standardized report structure element, in particular the corresponding standardized text segment. For example, the text segment may be marked by underlining or by changing a text property of the text segment, such as the colour or size of the text. For example, different text segment corresponding to different medical findings may be marked, e.g. underlined, in different colours. This embodiment may provide a simple and efficient means to let the user take note of a possible adjustment. The displayed standardized report structure element may, for example, be displayed next to the medical report, e.g. on a screen.

According to an embodiment, a confidence score describing the probability of the probable structured medical finding being correct is provided by the classification algorithm, and wherein the confidence score is displayed to the user, in particular in addition to the corresponding standardized text segment. For example, the output of a trained machine learning algorithm, such as a trained neural network, may comprise a confidence value describing the probability. Hence, in a simple example case, the class probability as provided by the classification algorithm, e.g. by a classification network of the classification algorithm, can be used as a confidence score. Advantageously, more comprehensive information may thus be provided to the user, enabling the user to more quickly assess, whether the proposed adaption makes sense and is likely correct. The confidence score may also be denoted as probability score.

Additionally or alternatively a refined list of structured medical findings may be provided comprising the at least one adjusted structured medical finding. Optionally, a probability score can be assigned towards the refined list towards the structured medical findings and/or towards the probable structured medical findings.

According to an embodiment, proposing an adjustment to a user comprises providing a hover functionality, wherein the proposed adjustment, in particular the standardized text segment, is displayed when the user moves a pointer over a text segment that is proposed to be adjusted. The hover functionality may provide a particularly time-efficient means to give the corresponding user the information what change is proposed. Advantageously, the user can directly access this information, e.g. by moving a pointer over the corresponding text segment. The hover functionality may be combined with a means to accept the proposed adaption. For example, a click button may be provided to the user to accept the proposed adaption. Additionally or alternatively, a corresponding decline option, e.g. a click button to decline, may be provided.

Additionally or alternatively a refined list of structured medical findings may be provided comprising the at least one adjusted structured medical finding. A hover over functionality may deployed, wherein the user hovers over the refined list, the system proposes possible improvements that can be made and allows the user to choose the set of refinements.

According to an embodiment, the adjustment of the medical report comprises one or more of the following: replacement, addition, and simplification of the one or several text segments according to the at least one standardized text segment.

According to another aspect of the invention, a computer program comprising instructions which, when the program is executed by a computer, cause the computer to carry out the method according to any one of the preceding claims. The instructions may comprise any of the method steps and their variants as described herein. The computer program may be stored on a data storage medium, in particular a non-volatile data storage medium.

According to another aspect of the invention, a computer system configured to execute the method as described herein is provided.

According to another aspect of the invention a user interface is provided. The user interface comprises a screen for providing a medical report to a user with marked text segments that are to be adjusted and a display functionality for showing the user a proposed standardized text segment as adjustment for the text segment of the medical report based on at least one structured medical finding. The structured medical finding may be a structured medical finding determined by a method as described herein. Optionally, a confidence score describing the probability of the structured medical finding being correct may be displayed to the user in addition to the corresponding standardized text segment. Optionally, the user interface may provide an input functionality, such as a menu option, regarding a selection of at least one language for the adjustments of the medical report. This input functionality may, for example, be useful in a tele-radiology set-up, where the radiologist and a physician receiving the report speak different languages.

These and other aspects of the invention will be apparent from and elucidated with reference to the embodiment(s) described hereinafter

BRIEF DESCRIPTION OF THE DRAWINGS

The invention shall now be illustrated by means of embodiments with reference to the attached drawings, in which:

Fig. 1 shows a flow diagram of a computer-implemented method for determining at least one structured medical finding in a medical report according to an embodiment of the invention;
Fig. 2 shows a flow diagram of a computer-implemented method for determining at least one structured medical finding in a medical report according to another embodiment of the invention;
Fig. 3 shows a flow diagram of a computer-implemented method for adjusting a medical report according to an embodiment of the invention; and
Fig. 4 shows a simplified representation of a user interface according to an embodiment of the invention.

DETAILED DESCRIPTION OF EMBODIMENTS

Throughout the figures, the same or corresponding features/elements of the various embodiments are designated with the same reference numbers.

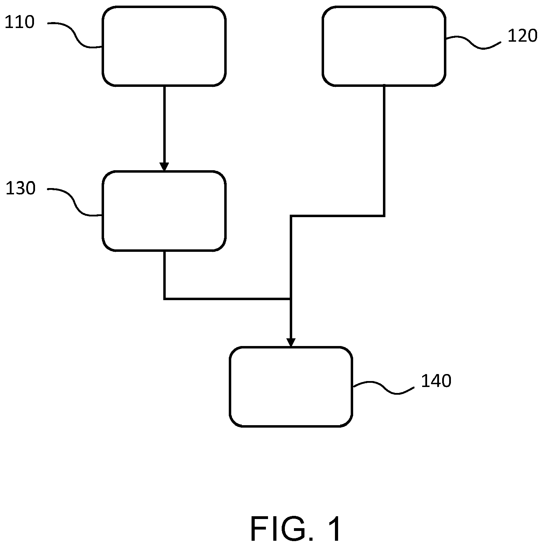

Fig. 1 shows a flow diagram of a computer-implemented method for determining at least one structured medical finding in a medical report according to an embodiment of the invention. In a first step 110, the medical report 1 to be adjusted is received. The medical report 1 comprises one or several text segments 6 describing medical findings. Furthermore 120, at least one medical image, on which the medical report 1 is based, is received. In a further step 130, a searching algorithm is applied to the medical report 1 in order to identify at least one medical finding in the medical report 1 and determine at least one corresponding structured medical finding. For example, the searching algorithm may be configured to search for keywords describing the medical findings in order to identify structured medical findings in the medical report 1. For example, in each text segment 6 of the medical report 1 the searching algorithm may search for the keywords and identify a medical finding if the corresponding keywords are found. Preferably, the searching algorithm may be configured to determine findings based on the structure of the structured findings. The structured medical findings may be structured to comprise multiple properties. Each property may comprise a list of options that may apply for that property. Preferably at least one of the properties describes an anatomic position of the medical finding and at least one of the properties describes a medical abnormality. The keywords used by the searching algorithm may be options of properties of the structured medical findings described in text form or synonyms thereof.

Table 1 shows an exemplary excerpt of a corresponding structure that may be applied in the context of the invention's structured medical findings. The heading of the table 1 shows multiple properties describing anatomic positions and medical abnormalities and the columns comprise multiple options for each property. The properties in this example are: compartment, sub-compartment, type of structure, sub-structure, subsub-structure(s), pathology, grading, and further properties. In this exemplary excerpt, an overview over meniscus and cartilage related medical findings in the knee is shown. Cartilage and meniscus defects are among the most common findings in knee examinations, in particular knee magnetic resonance imaging examinations, as the cartilage and the menisci degrade with age. Menisci may be graded according to internationally standardized grading schemes, such as the Stoller scheme. According to Stoller, grade I refers to focal hyperintensities which do not reach the surface. Grade II refers to line-shaped hyperintensities, reaching the surface in maximally one image / slice. Grade III refers to a hyperintensity which reaches at least one surface on at least two consecutive images / slices. Typically, a "meniscus tear" may refer to Stoller grade III (Stoller 3). Hence, in the structure according to this table, further properties are given for Stoller 3. Abnormal cartilage may be described using internationally standardized grading schemes such as Valloton or ICRS for assessing the severity of the cartilage damage. A complete set of medical findings for the knee may cover more medical findings, for example including medical findings related to bones and ligaments. Generally, a corresponding table may also be devised analogously for other parts of the human body. The table is structured from left to right corresponding to a sequence of the properties and the right columns depend on the left columns, such that options chosen for the properties on the left affect the choice of options of properties farther on the right. For example, the subsub-structure options "anterior horn", "pars intermedia" and "posterior horn" are only available for the type of structure being "meniscus". Generally, on the left, the properties describe a more general anatomic position (e.g., the general compartment) and, going to the right, more specific anatomic positions are described (sub-compartment, type of structure, sub-structure etc.) depending on the more general anatomic positions.

In a further step 140, a classification algorithm is applied to the medical image. In some cases, the classification may only be applied when a structured medical finding determined by the searching algorithm is ambiguous. An ambiguity may comprise a missing property of a structured medical finding in the medical report 1 or contradicting properties or options of properties. The classification algorithm is used to verify and/or complement the structured medical finding and the output of the classification algorithm is at least one probable structured medical finding. If applicable, e.g. if new properties are found, an error is discovered, or an ambiguity is resolved, the structured medical finding determined by the searching algorithm is adjusted on the basis of the output of the classification algorithm. For example, a missing property may be added to the structured medical finding. The classification algorithm may be configured to determine the most likely missing property and to add the most likely missing property to the structured medical finding.

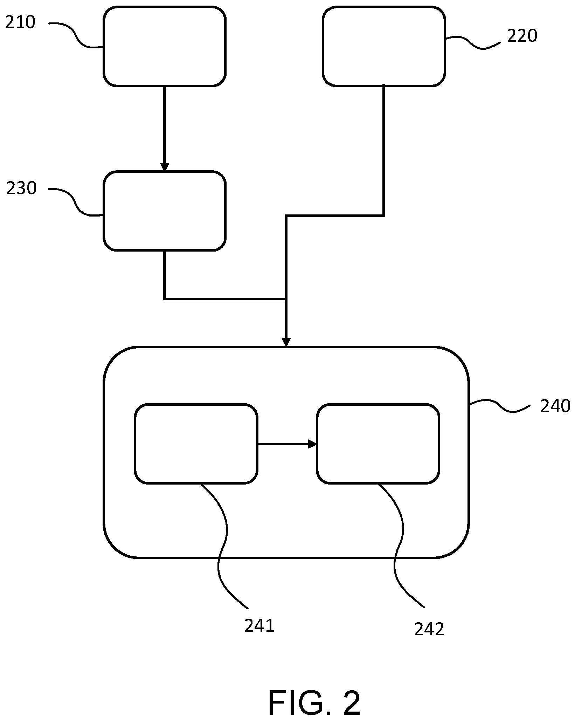

Fig. 2 shows a flow diagram of a computer-implemented method for determining at least one structured medical finding in a medical report 1 according to another embodiment of the invention. In a first step 210, the medical report 1 to be adjusted is received. The medical report 1 comprises one or several text segments 6 describing medical findings. Furthermore 220, at least one medical image, on which the medical report 1 is based, is received. In a further step 230, a searching algorithm is applied to the medical report 1 in order to identify at least one medical finding in the medical report 1 and determine at least one corresponding structured medical finding. The searching algorithm and the structured medical findings may be analogous to the searching algorithm and structured medical findings as described with respect to Fig. 1. In a further step 240, a classification algorithm is applied to the medical image. In some cases, the classification may only be applied when a structured medical finding determined by the searching algorithm is ambiguous. The classification algorithm is used to verify and/or complement the structured medical finding and the output of the classification algorithm is at least one probable structured medical finding. If applicable, e.g. if new properties are found, an error is discovered, or an ambiguity is resolved, the structured medical finding determined by the searching algorithm is adjusted on the basis of the output of the classification algorithm. The classification algorithm comprises an anatomical segmentation model that segments image information in the medical image into anatomical parts in a sub-step 241. In a further sub-step 242, based on the segmented anatomical parts, regions of interest are defined that are most likely to contain medical findings and the regions of interest are used to detect and determine structured medical findings in the medical image.

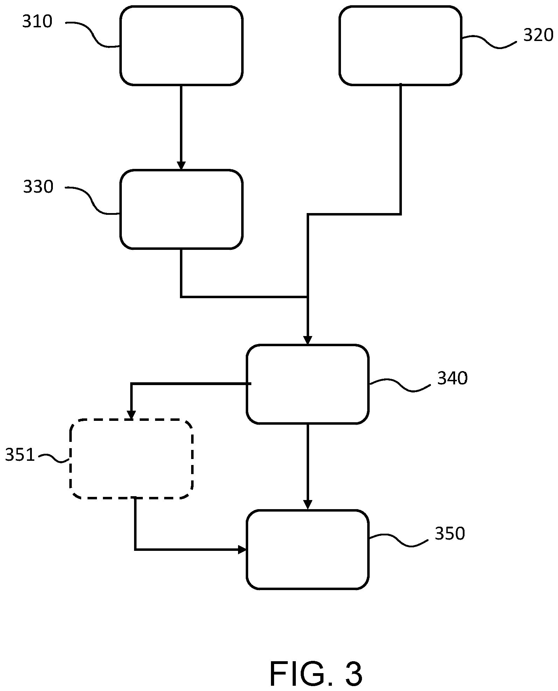

Fig. 3 shows a flow diagram of a computer-implemented method for adjusting a medical report 1 according to an embodiment of the invention. In a first step 310, the medical report 1 to be adjusted is received. The medical report 1 comprises one or several text segments 6 describing medical findings. Furthermore 320, at least one medical image, on which the medical report 1 is based, is received. In a further step 330, a searching algorithm is applied to the medical report 1 in order to identify at least one medical finding in the medical report 1 and determine at least one corresponding structured medical finding. The searching algorithm and the structured medical findings may be analogous to the searching algorithm and structured medical findings as described with respect to Fig. 1 or Fig. 2. In a further step 340, a classification algorithm is applied to the medical image. The classification algorithm may be provided analogously to the classification algorithm and as described with respect to Fig. 1 or Fig. 2. In a further step 350, an adjustment of the medical report 1 based on the structured medical findings is performed automatically or proposed to a user, e.g. via a user interface. The adjustment may be based on a report structure source. In an additional step 351, at least one standardized text segment 2 corresponding to the structured medical finding may be retrieved from the standardized report structure source. In this case, performing or proposing to a user an adjustment of the medical report 1 is based on the at least one standardized text segment 2. The standardized text resource may comprise standardized text segments 2 in a plurality of languages for each structured medical finding. In this case the user may be given the option to select at least one language for the adjustments of the medical report 1 in a user interface. Proposing an adjustment to a user may comprise marking a text segment 6 in the medical report 1, in particular a text segment 6 corresponding to an ambiguous structured medical finding, and displaying to the user the corresponding standardized text segment 2, e.g. in an additional window of the user interface. Proposing an adjustment to the user my comprise providing a hover functionality and the proposed adjustment may be displayed when the user moves a pointer 4 over a text segment 6 that is proposed to be adjusted. In addition to the standardized text segment 2, a confidence score 3 may be displayed to the user. The confidence score 3 may describe the probability of whether the probable structured medical finding is correct and may thus help the user in the decision of whether the adaption is likely advisable.

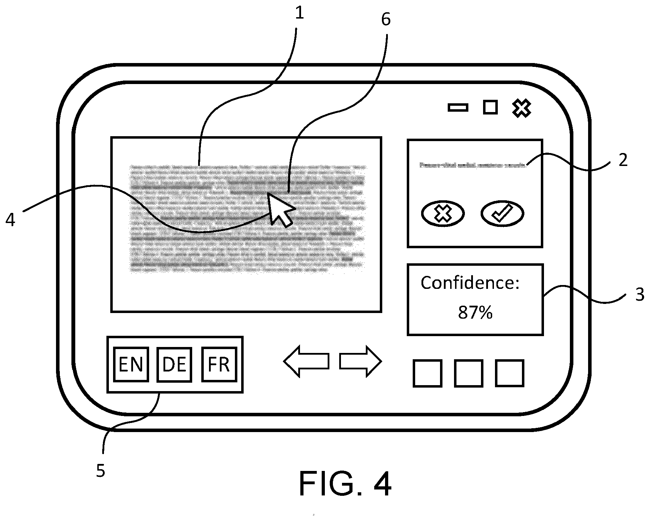

Fig. 4 shows a simplified representation of a user interface according to an embodiment of the invention. The user interface comprises a window where the medical report 1 or parts of the medical report 1 are presented to the user. Where applicable, an adjustment of the medical report 1 is proposed to the user based on the most probable structured medical findings as determined in a method according to the invention. In this example, proposing an adjustment to a user comprises marking a text segment 6 in the medical report 1. When hovering over the marked text segment 6 with a pointer 4 a proposed standardized text segment 2 based on a determined probable structured medical finding is displayed to the user and the user has the options to accept or decline the adjustment. In addition, a confidence score 3 describing the probability of the probable structured medical finding being correct is displayer to the user in in addition to the corresponding standardized text segment 2. Furthermore, the user is given the option of a language selection 5, via which the user may select a language for the adjustments of the medical report 1. Optionally, the medical report 1 may be translated into this language.

The above-discussion is intended to be merely illustrative of the present system and should not be construed as limiting the appended claims to any particular embodiment or group of embodiments. Accordingly, the specification and drawings are to be regarded in an illustrative manner and are not intended to limit the scope of the appended claims. Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "an" or "a" does not exclude a plurality. A single processor or other unit may fulfil the functions of several items recited in the claims. The mere fact that certain measures are recited in mutually different dependent claims does not indicate that a combination of these measured cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

REFERENCE SIGNS

| | |
|---|---|
| 1 | medical report |
| 2 | standardized text segment |
| 3 | confidence score |
| 4 | pointer |
| 5 | language selection |
| 6 | text segment of the medical report |
| 110-140 | method steps |
| 210-242 | method steps |
| 310-350 | method steps |

Table I

| compartment | sub-compartment | type of structure | sub-structure | subsub-structure(s) | pathology | grading | further properties |
|---|---|---|---|---|---|---|---|
| ID | ID | ID | ID | Set of IDs | ID | ID | ID |
| femoro-tibial | medial, lateral | meniscus | (not used) | anterior horn, pars intermedia, posterior horn | (not used) | Stoller 1, Stoller 2, Stoller 3, | only for Stoller 3: vertical, radial, horizontal, bucket- |
| femoro-tibial | medial, lateral | meniscus | (not used) | (not used) | (not used) | Watanabe 1, Watanabe 2, | (not used) |
| femoro-tibial | medial, lateral | meniscus | (not used) | (not used) | vacuole, other | (not used) | (not used) |

(continued)

| compartment | sub-compartment | type of structure | sub-structure | subsub-structure(s) | pathology | grading | further properties |
|---|---|---|---|---|---|---|---|
| femoro-tibial | medial, lateral | cartilage | femoral | segment 1, segment 2, ..., segment 5 | (not used) | ICRS/Valloton 1, ICRS/Valloton 3, ICRS/-Valloton 4, | (not used) |
| | | | tibial | segment 1, segment 2, | | | |
| femoro-patellar | (not used) | | (not used) | retropatellar, trochlear | | | |
| femoro-tibial | medial, lateral | cartilage | femoral, tibial | (not used) | other | (not used) | (not used) |
| femoro-patellar | (not used) | | (not used) | | | | |

**Claims**

1. A computer-implemented method for determining at least one structured medical finding in a medical report (1), the method comprising the following steps:

   (a) receiving the medical report (1), wherein the medical report (1) comprises one or several text segments (6) describing medical findings, and receiving at least one medical image on which the medical report (1) is based;
   (b) applying a searching algorithm to the medical report (1) in order to identify at least one medical finding in the medical report (1) and determine at least one corresponding structured medical finding;
   (c) at least in case the determination of a structured medical finding by the searching algorithm fulfils a pre-defined ambiguity criterion, applying a classification algorithm to the medical image to verify and/or complement the structured medical finding, wherein the output of the classification algorithm is at least one probable structured medical finding, and adjusting the structured medical finding determined by the searching algorithm on the basis of the output of the classification algorithm.

2. The method according to claim 1,

   wherein the classification algorithm comprises an anatomical segmentation model that segments image information in the medical image into anatomical parts,
   wherein the classification algorithm detects and determines probable structured medical findings based on the segmented anatomical parts.

3. The method according to claim 2,

   wherein, based on the segmented anatomical parts, regions of interest are defined that are most likely to contain medical findings,
   wherein the regions of interest are used to detect and determine structured medical findings in the medical image.

4. The method according to any one of the preceding claims,

   wherein the structured medical findings are structured to comprise multiple properties, at least one of the properties describing an anatomic position of the medical finding and at least one of the properties describing a medical abnormality,
   wherein, optionally, each property comprises a list of options of possible abnormalities that may apply for that property.

5. The method according to claim 4,
   wherein the multiple properties comprise a sequence of properties, wherein at least one of the properties determines at least one following property and/or the list of options for a following property in the sequence.

6. The method according to any one of claims 4 to 5,

   wherein the ambiguity criterion comprises a missing property of a structured medical finding in the medical report (1),
   wherein the classification algorithm is configured to determine and add the missing property to the structured medical finding.

7. The method according to any one of the preceding claims,
   wherein the searching algorithm is configured to search for specific text segments, in particular keywords, describing the medical findings in order to identify structured medical findings in the medical report (1), wherein in particular the keywords are options of properties of the structured medical findings described in text form or synonyms thereof.

8. The method according to any one of the preceding claims,
   wherein at least one identifier is stored together with the medical report (1) that comprises information about the at least one structured medical finding.

9. A computer-implemented method for adjusting a medical report (1) towards a standardized medical report (1) format, the standardized medical report (1) format comprising standardized text segments (2) describing medical findings, the method comprising the following steps:

   - carrying out the method according to any one of the preceding claims;
   - automatically performing or proposing to a user an adjustment of the medical report (1) based on the structured medical finding.

10. The method according to claim 9,

    wherein at least one standardized report structure element, in particular comprising at least one standardized text segment (2) and/or at least one standardized object including graphics, corresponding to the structured medical finding is retrieved from a standardized report structure source,
    wherein performing or proposing to a user an adjustment of the medical report (1) is based on the at least one standardized report structure element.

11. The method according to any one of claims 9 to 10,
    wherein proposing an adjustment to a user comprises marking a text segment (6) in the medical report (1), in particular a text segment (6) corresponding to an ambiguous structured medical finding, and displaying to the user the corresponding standardized text segment (2).

12. The method according to any one of claims 9 to 11,

    wherein a confidence score (3) describing the probability of the probable structured medical finding being correct is provided by the classification algorithm,
    and wherein the confidence score (3) is displayed to the user, in particular in addition to the corresponding standardized text segment (2).

13. The method according to any one of claims 9 - 12,
    wherein the standardized text resource comprises standardized text segments (2) in a plurality of languages for each structured medical finding, and wherein the method comprises receiving user input regarding a selection of at least one language for the adjustments of the medical report (1).

14. A computer program comprising instructions which, when the program is executed by a computer, cause the computer to carry out the method according to any one of the preceding claims.

15. A computer system configured to execute the method according to any one of the claims 1 to 13.

110

120

130

140

FIG. 1

FIG. 2

FIG. 3

FIG. 4

Europäisches
Patentamt

European
Patent Office

Office européen
des brevets

# EUROPEAN SEARCH REPORT

Application Number

EP 23 20 8722

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2022/253592 A1 (RAO SHANKAR [US] ET AL) 11 August 2022 (2022-08-11) | 1-5,7-15 | INV.<br>G16H15/00 |
| A | * figures 1-17Y *<br>* page 1, paragraph 0003 – page 21, paragraph 0161 *<br>* page 40, paragraph 0302 – page 62, paragraph 0438 * | 6 | G16H50/20<br><br>ADD.<br>G16H30/40 |
| X | US 2021/264212 A1 (PAIK DAVID SEUNGWON [US] ET AL) 26 August 2021 (2021-08-26) | 1-5,7-15 | |
| A | * page 1, paragraph 0002 – page 7, paragraph 0061 *<br>* page 16, paragraph 0128 – page 23, paragraph 0182 *<br>* page 26, paragraph 0204 – page 32, paragraph 0247 *<br>* figures 1-34 * | 6 | |
| X | US 2021/313045 A1 (WU JOY TZUNG-YU [US] ET AL) 7 October 2021 (2021-10-07) | 1-5,7-15 | |
| A | * figures 1-5 *<br>* page 1, paragraph 0004 – page 5, paragraph 0039 *<br>* page 7, paragraph 0054 – page 12, paragraph 0084 *<br>* page 15, paragraph 0106 – paragraph 0107 * | 6 | TECHNICAL FIELDS SEARCHED (IPC)<br><br>G16H |

The present search report has been drawn up for all claims

| Place of search | Date of completion of the search | Examiner |
|---|---|---|
| Munich | 25 March 2024 | Sasa Bastinos, Ana |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons

& : member of the same patent family, corresponding
   document

EPO FORM 1503 03.82 (P04C01)

**EP 4 553 842 A1**

## ANNEX TO THE EUROPEAN SEARCH REPORT
## ON EUROPEAN PATENT APPLICATION NO.

**EP 23 20 8722**

This annex lists the patent family members relating to the patent documents cited in the above-mentioned European search report.
The members are as contained in the European Patent Office EDP file on
The European Patent Office is in no way liable for these particulars which are merely given for the purpose of information.

25-03-2024

| Patent document cited in search report | Publication date | Patent family member(s) | | Publication date |
|---|---|---|---|---|
| US 2022253592 A1 | 11-08-2022 | NONE | | |
| US 2021264212 A1 | 26-08-2021 | AU | 2020357886 A1 | 21-04-2022 |
| | | CA | 3156519 A1 | 08-04-2021 |
| | | EP | 4038622 A1 | 10-08-2022 |
| | | US | 2021216822 A1 | 15-07-2021 |
| | | US | 2021264212 A1 | 26-08-2021 |
| | | WO | 2021067624 A1 | 08-04-2021 |
| US 2021313045 A1 | 07-10-2021 | NONE | | |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **MICHAEL P. HARTUNG ; IAN C. BICKLE ; FRANK GAILLARD ; JEFFREY P. KANNE**. How to Create a Great Radiology Report. *RadioGraphics*, 2020, vol. 40 (6), 1658-1670